(19) **Europäisches Patentamt / European Patent Office / Office européen des brevets**

(11) **EP 2 583 887 B1**

(12) **EUROPEAN PATENT SPECIFICATION**

(45) Date of publication and mention
of the grant of the patent:
**21.09.2016 Bulletin 2016/38**

(51) Int Cl.:
**B62J 99/00** *(2009.01)*　　**A61B 5/22** *(2006.01)*

(21) Application number: **10853246.6**

(86) International application number:
**PCT/JP2010/060312**

(22) Date of filing: **17.06.2010**

(87) International publication number:
**WO 2011/158365 (22.12.2011 Gazette 2011/51)**

(54) **WORK RATE MEASUREMENT DEVICE**

VORRICHTUNG ZUR MESSUNG DES ARBEITSDURCHSATZES

DISPOSITIF DE MESURE DU TAUX DE TRAVAIL

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(43) Date of publication of application:
**24.04.2013 Bulletin 2013/17**

(73) Proprietor: **Pioneer Corporation
Kawasaki-shi, Kanagawa 212-0031 (JP)**

(72) Inventors:
• **FUJITA Ryujiro
Kawasaki-shi, Kanagawa 212-0031 (JP)**

• **KODAMA Yasuteru
Kawasaki-shi, Kanagawa 212-0031 (JP)**

(74) Representative: **Sajda, Wolf E. et al
Meissner Bolte Patentanwälte
Rechtsanwälte Partnerschaft mbB
Postfach 86 06 24
81633 München (DE)**

(56) References cited:
**WO-A2-02/081257　　JP-A- 2 299 992
JP-A- 7 151 620　　JP-A- 2000 140 415**

**Description**

Technical Field

The present invention relates to a power measurement apparatus.

Background Art

The number of people who ride bicycles is increasing due to the increased health awareness in recent years. The people who ride bicycles include ones who enjoy cycling moderately as a hobby and ones who ride bicycles for exercise seriously. In a case of cycling for exercise, for example, the calories-out of a person who is riding a bicycle, that is, the work on a bicycle performed by the cyclist on the bicycle is attracting attention as one indicator.

There are many methods for calculating work. For example, it is possible to calculate work by multiplying power that is work done per unit time. Conventionally, there have been many apparatuses for measuring power (see Patent Literature 1).

Citation List

Patent Literature

PTL1: Japanese Patent Application Laid-Open Publication JP-A-7-151 620.
PTL2: Patent Document WO 02/081257 A2 (VOSS DARELL, 17-10-02).

Summary of the Invention

Technical Problem

The power measurement apparatus disclosed in Patent Literature 1, and in Patent Literature 2 according to the respective preambles of claims 1 and 2, (hereinafter called "prior art") has a function to measure the power of the work done on the pedals. With the prior art, the rate of rotation of the pedals which is changing, the acceleration of a bicycle in the direction in which the bicycle is running, and the velocity of the wind coming toward the running bicycle are detected to calculate the power of the cyclist.

With the prior art, although the air resistance is reflected in the power by detecting the velocity of the wind coming toward the running bicycle, the frontal projected area of a part subject to air resistance, which highly depends on air resistance, is not reflected in the power. Therefore, the accuracy of the calculated power is reduced.

In view of the background set forth in the preceding paragraphs, it is therefore an object of the present invention to provide a power measurement apparatus configured to prevent such a loss of accuracy.

Solution to the Problem

To solve the above-described problems, the present invention provides the power measurement apparatus that measures power of work done on a bicycle, comprising a calculating part. The calculating part detects a change in a projected area of a mobile object, the projected area being projected on a plane of the mobile object perpendicular to a ground, the mobile object including at least a cyclist on the bicycle and moving along the ground. The calculating part calculates the projected area in which the change is reflected.

In addition, to solve the above-described problem, the present invention provides a program that allows a computer to function as a calculating means for detecting a change in a projected area of a mobile object, the projected area being projected on a plane of the mobile object perpendicular to a ground, the mobile object including at least a cyclist on a bicycle and moving along the ground, and for calculating the projected area in which the change is reflected.

Brief Description of Drawings

FIG. 1(a)    is a side view showing a bicycle to which a power measurement apparatus is attached;
FIG. 1(b)    is an enlarged view showing the part of the bicycle to which the power measurement apparatus is attached;

FIG. 2(a)    is a front view showing the power measurement apparatus;

FIG. 2(b)    is a rear view showing the power measurement apparatus;

FIG. 2(c)    is a longitudinal sectional view showing the power measurement apparatus;

FIG. 2(d)    is a cross-sectional view of FIG. 2(c) taken along line A-A;

FIG. 3    is a block diagram showing the electrical system of the power measurement apparatus;

FIG. 4(a)    is a side view showing a cyclist on the bicycle, stretching his arms;

FIG. 4(b)    is a front view of FIG. 4(a);

FIG. 5(a)    is a side view showing the cyclist on the bicycle, bending his arms;

FIG. 5(b)    is a front view of FIG. 5(a);

FIG. 6    is a block diagram showing the functional structure of the power measurement apparatus;

FIG. 7    is a flowchart showing the steps in processing of power measurement;

FIG. 8    is a flowchart showing the steps in processing to calculate a frontal projected area;

FIG. 9    is a flowchart showing the steps in processing of power calculation; and

FIG. 10    is a drawing showing a conversion graph of the frontal projected area.

Description of Embodiments

Embodiment 1

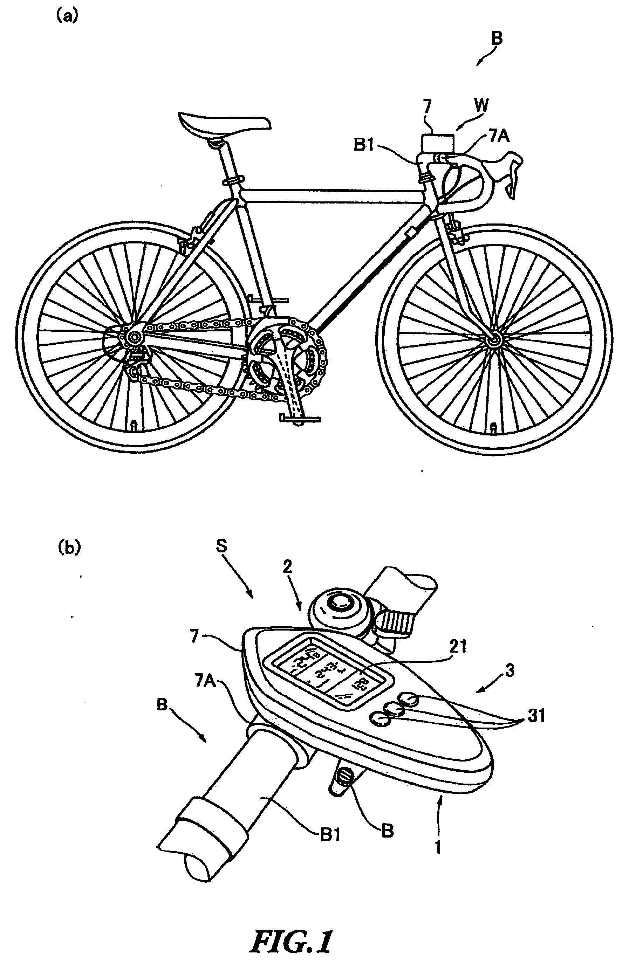

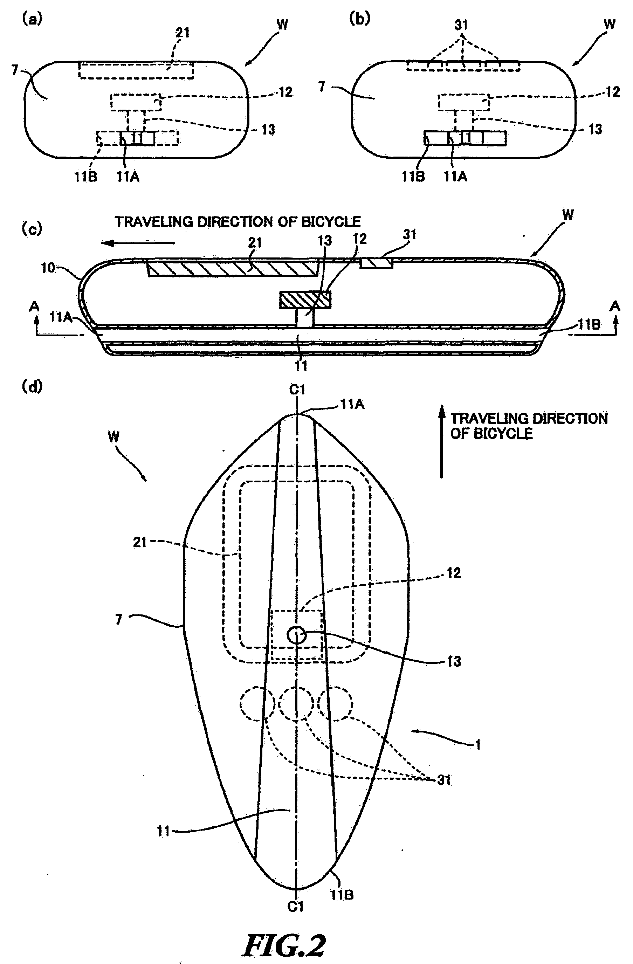

Now, the present invention according to an embodiment will be described in detail with reference to the drawings. FIG. 1 is an external view showing a bicycle equipped with a cycle computer S that serves as a power measurement apparatus W according to the present invention. FIG. 2 is a drawing showing an example of the structure of the cycle computer S, or the power measurement apparatus W.

The power measurement apparatus W includes: a wind velocity measurement device 1 that measures a wind velocity relative to a bicycle B; a display device 2 that displays predetermined information that is set in advance, in addition to the power of the cyclist on the bicycle B; an input device 3 that is operated to input predetermined information; a communication device 4 that can communicate with a predetermined external device (not shown); a sensor group 5 that measures predetermined information; a control device 6 that that controls each device; and a main body 7 that accommodates those devices and part of the sensor group and integrates them.

The main body 7 is made of synthetic resin such as plastic and shaped like an approximately rectangular solid. An attachment part 7A to be attached to the bicycle B is provided in one face of the main body 7. The attachment part 7A is made of synthetic resin such as plastic, and configured to be fixed to a handle B1 while the cyclist is holding the handle B1.

On the other face (hereinafter called "front face") opposite to the face on which the attachment part 7A is provided (hereinafter called "rear face"), a display part 21 of the display device 2 on which predetermined information is actually displayed, an input part 31 of the input device 3 that the cyclist can operate, and a distance measurement sensor 52 belonging to the sensor group 5 are provided.

When the power measurement apparatus W is attached to the handle B1 via the attachment part 7A, the rear face of the main body 7 faces the ground while the front face of the main body 7 faces the atmosphere. Therefore, the cyclist can easily see and check the display part 21 of the display device 2 and the input part 31 of the input device 3.

Here, with the present embodiment, the front face and the rear face of the main body 7 are formed in parallel with one another, and are the same in shape and size. The shape is an approximate ellipse, or an approximate diamond having the diagonal lines of different lengths. When the power measurement apparatus W is appropriately attached to the handle B1 of the bicycle B, the longitudinal direction of those faces is the same as the traveling direction of the bicycle.

Then, the display part 21 and the input part 31 are arranged side by side in the longitudinal direction. When the power measurement apparatus W is appropriately attached to the handle B1 of the bicycle B, the display part 21 is placed before the input part with respect to the bicycle B.

The wind velocity measurement device 1 is provided between the attachment part 7A and the front face on which the display part 21 and the input part 31 are arranged. The wind velocity measurement device 1 includes: a flow passageway 11 that allows an airflow such as wind to flow through; a sound pressure sensor 12 that detects the sound pressure of a sound wave (vibration); and a detection port 13 that generates a sound wave (vibration) from the airflow in the upstream side of the sound pressure sensor 12.

The flow passage 11 is formed by a hole that runs through the main body 7 in the longitudinal direction. That is, the hole constituting the flow passage 11 penetrates the main body 7 from end to end. Then, the opening formed in the end surface of the power measurement apparatus W in the front side of the bicycle B (in the traveling direction) forms an inflow port 11A, and the opening formed in the end surface of the power measurement apparatus W in the rear end side of the bicycle B (in the direction opposite to the traveling direction) forms an outflow port 11B. Therefore, the airflow such as wind that is coming from the forward and backward sides of the bicycle B flows from the inflow port 11A and flows out from the outflow port 11B.

The flow passage 11 is entirely surrounded by the main body 7 in the circumferential direction with respect to its central axis C1 (in the longitudinal direction). Therefore, the flow passage 11 is shield from the airflow such as wind that is coming from a lateral direction (the direction orthogonal to the central axis C1) of the bicycle B to the bicycle B.

The cross-section orthogonal to the central axis C1 of the flow passage 11 (hereinafter called "the cross-section of the flow passage 11") has an approximately rectangular shape, and gradually widens from the front side to the back side of the bicycle B. That is, the outflow port B is wider than the inflow port 11A.

Here, with the present embodiment, the height of the flow passage 11 (that is, the length of the flow passageway 11 orthogonal to the front and rear faces) is fixed, but only the width of the flow passage 11 (the length of each of the front and rear faces in the minor axis direction) increases from the front side to the back side of the bicycle B. The central axis of the flow passage 11 is parallel to the faces of the main body 7.

Here, with Embodiment 1, although the cross-section of the flow passage 11 is shaped like an approximate ellipse or an approximate diamond, the shape of the cross-section of the flow passage 11 is not limited unless otherwise noted. In addition, with the present embodiment, although the length of the flow passage 11 increases only in the minor axis direction of the faces of the main body 7 from the front side to the back side of the bicycle B, how the cross-section of the flow passage 11 widens is not limited as long as the cross-section widens from the front side to the back side. Moreover, with the present embodiment, although the central axis C1 of the flow passage 11 is a straight line parallel to the faces of the main body 7, a straight line tilted to the faces, or a curved line is possible.

The sound pressure sensor 12 is provided in the middle of the main body 7 in the longitudinal direction. That is, the sound pressure sensor 12 is provided between the front face of the main body 7 and the flow passage 11. The structure and the shape of the sound pressure sensor 12 are not limited as long as they allow the sound pressure sensor 12 to detect the sound pressure of a sound wave. With the present embodiment, a MEMS microphone packaged as a flat plate is used as the sound pressure sensor 12.

In addition, the detection port 13 is formed from the hole wall of the flow passage 11 to a port of the sound pressure sensor 12 to collect sound waves (not shown, and hereinafter referred to as "sound port"), and therefore allows communication between the flow passage 11 and the sound port of the sound pressure sensor 12. Therefore, when the airflow such as wind flowing into the flow passage 11 passes through the detection port 13, a turbulent flow (so-called "wind noise") is generated.

Since this turbulent flow moves toward the sound pressure sensor 12 through the detection port 13, the sound pressure sensor 12 detects the turbulent flow generated due to an airflow such as wind, as a sound wave (so-called "wind noise"). The sound pressure sensor 12 converts the detected sound pressure into an electrical signal and outputs the signal to the control device 6 described later. Here, although the shape of the detection port 13 is not limited, it is preferred that the detection port 13 has a column shape because it is possible to prevent the attenuation of a sound wave (vibration) due to turning loss.

Here, when a hollow structure having both opening ends is disposed in the atmosphere, the following facts are experimentally or empirically known as the behavior of an airflow. In a case in which the cross-section of the cavity of a hollow structure widens to the direction in which the airflow flows (hereinafter called "case 1"), the velocity of the airflow increases at the inlet of the hollow structure and decreases in the direction of the outlet. On the other hand, in a case in which the cavity of a hollow structure narrows along the direction in which the airflow flows (hereinafter called "case 2"), the velocity of the airflow decreases at the inlet and increases in the direction of the outlet.

Here, among the locations near the inlet and the outlet of the cavity of the hollow structure, the location just before the inlet and the location just after the outlet are in the atmosphere and under the same atmospheric pressure. Therefore, the velocity of the air flow just before flowing into the cavity of the hollow structure is the same between case 1 and case 2, and the velocity of the airflow just after flowing out from the cavity of the hollow structure is also the same between case 1 and case 2.

Moreover, the velocity of the airflow just before flowing into the cavity is the same as the velocity of the airflow just after flowing out from the cavity. That is, the tendency of the velocity of the airflow in the cavity varies depending on whether the cross-section of the cavity increases or decreases along the direction in which the airflow flows.

According to this tendency, when, for example, the cross-section orthogonal to the central axis C1 of the flow passage 11 widens from the front side to the back side of the bicycle B as in the present embodiment, the directivity of the sound pressure sensor 12 is higher in the direction from the back side to the front side of the bicycle B (the traveling direction of the bicycle B) than in the direction from the front side to the back side of the bicycle B (the direction opposite to the traveling direction). Now, the reason will be explained.

With the above-described example of the hollow structure, the pressure in the cavity just after the outlet is the same as the atmospheric pressure in both cases (case 1 and case 2), and therefore the pressure in the cavity gradually approaches the atmospheric pressure toward the outlet, and finally converges on the atmospheric pressure at the outlet. Therefore, the velocity of the airflow just before the outlet is the same between case 1 and case 2.

Meanwhile, although the pressure just before the inlet is the same as the atmospheric pressure, the velocity of the airflow just after the inlet is higher than the velocity of the airflow in the atmosphere (the outside of the cavity) because the velocity of the airflow increases just after the inlet in case 1. Meanwhile, in case 2, the velocity of the airflow just after the inlet is lower than the velocity of the airflow in the atmosphere because the velocity of the airflow decreases just after the inlet.

Here, with the present embodiment, when this example is applied to the airflows flowing through the flow passage 11, the velocity of the air flow flowing into the flow passage 11 from the front side of the bicycle B is higher than that of the air flow flowing into the flow passage 11 from the back side of the bicycle B at the detection port 13, because the inflow port 11A is narrower than the outflow port 11B, even if both of the air flows come to the bicycle B at the same velocity. Therefore, the directivity of the sound pressure sensor 12 is higher in the direction in which the airflow flows to the bicycle B from the front side of the bicycle B than in the direction in which the airflow flows to the bicycle B from the back side of the bicycle B.

Here, the wind velocity measurement device 1 measures a wind velocity based on the sound pressure detected by the sound pressure sensor 12. As described later, a wind velocity is measured by the control device 6 formed by a substrate on which a CPU 61 and a memory 62 are mounted. This control device 6 (not shown) is accommodated in the main body 7 and electrically connected to the sound pressure sensor 12 via a predetermined cable.

With the present embodiment, also the display part 21 and the input part 31 are electrically connected to the control device 6. The control device 6 performs predetermined display control and input control.

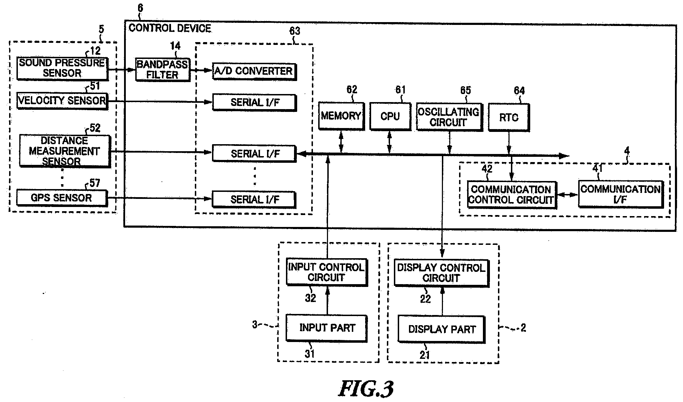

FIG. 3 is a block diagram showing the electrical system of the power measurement apparatus W according to Embodiment 1 of the present invention.

The display device 2 includes the display part 21 on which preset or predetermined information is displayed, and a display control circuit 22 that controls the display on the display part 21 according to a command from the CPU 61 described later. Here, the display part 21 and the display control circuit 22 are electrically connected to one another via a predetermined bus. In addition, with the present embodiment, the display part 21 is formed by a liquid crystal panel.

The input device 3 can be operated to input predetermined information, and has the input part 31 that outputs a predetermined input signal and an input control circuit 32. The input control circuit 32 performs input control based on the input signal from the input part 31 and outputs an input information signal indicating input information. Here, the input part 31 and the input control circuit 32 are electrically connected to one another via a predetermined bus. In addition, with the present embodiment, the input part 31 has a composite structure in which a numerical keypad and buttons are integrated.

By operating the input part 31, a command to start/end measuring predetermined information from the sensor group 5 and a command to start/end measuring power from the control device 6 are input, and also data indicating a user ID and predetermined conditions, such as information on the body of the user and information on the bicycle is input. When operated, the input part 31 transmits an input signal corresponding to the operation manner to the input control circuit 32, and then the input control circuit 32 outputs an input information signal indicating input information to the control device 6.

Here, with the present embodiment, although the input part 31 is constituted by three buttons arranged on the front face of the main body 7 in the minor axis direction, the configuration of the input part 31 is not limited to this, but a pointing device such as a track ball, a joystick and so forth are possible. In addition, another configuration is possible where the input part 31 is formed as a touch panel and integrated with the display part 21.

The communication device 4 includes a communication interface (I/F) 41 and a communication control circuit 42. The communication interface (I/F) 41 transmits and receives various data to and from an external device (not shown), for example, a mobile terminal such as a mobile phone or a fixed terminal such as a personal computer installed in a home. The communication control circuit 42 controls the communication interface 41 according to a command from the CPU 61 described later. The communication interface 41 and the communication control circuit 42 are electrically connected to one another via a predetermined bus.

The communication control circuit 42 controls the output of various data stored in the memory 62 to a predetermined external device (not shown) via the communication interface 41 according to the CPU 61, and also controls the input of the data from the predetermined external device via the communication interface 41. Here, the communication interface 41 is realized by various antennas when it is a wireless communication interface. Meanwhile, the communication interface 41 is realized by a terminal connected to a LAN cable and so forth when it is a wire communication interface.

As shown in FIG. 3, the sensor group 5 of the power measurement apparatus W includes a velocity sensor 51, distance measurement sensor 52, an inclination sensor 53, an atmospheric pressure sensor 54, a temperature sensor 55, a humidity sensor 56 and a GPS sensor 57. These sensors are appropriately attached to the inside or the outside of the main body 7 of the power measurement apparatus W depending on respective uses.

In addition, these sensors are electrically connected to an A/D converter or a serial I/F installed in the control device 6 via a wireless communication system or a wire communication system. The sensors 51 to 56 convert a measured value into an electrical signal and output the signal. The GPS sensor 57 receives a signal transmitted from a satellite and transmits the signal to the control device 6.

The velocity sensor 51 is a device that measures the absolute velocity of the bicycle B. The velocity sensor 51 includes, for example, a magnet fixed to a spoke and so forth of a wheel of the bicycle B and a magnet detector mounted on a chain stay and so forth. The velocity sensor 51 measures the number of times the magnet detector detects the magnet per unit time and outputs velocity data as an electrical signal into which the measured value is converted, to the control device 6. Then, the control device 6 multiplies this number of times by the outer circumference of the tire to calculate the velocity of the bicycle B.

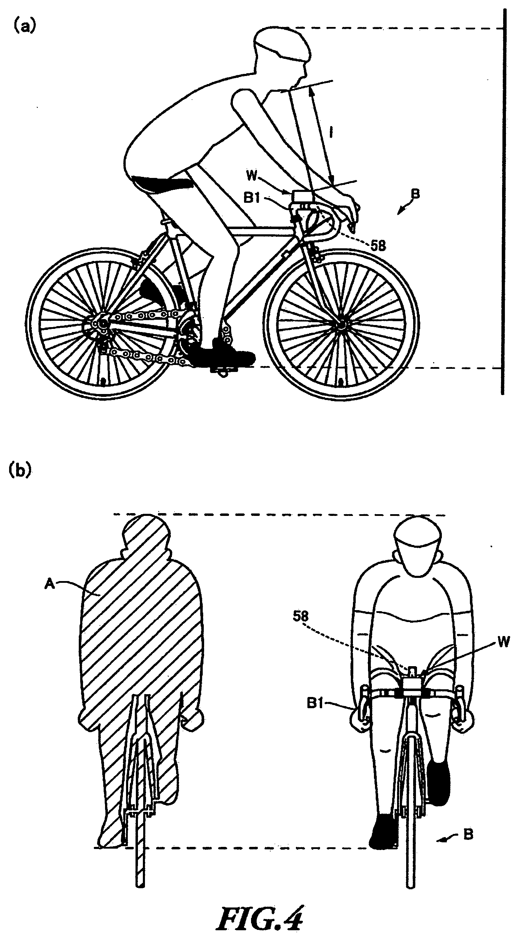

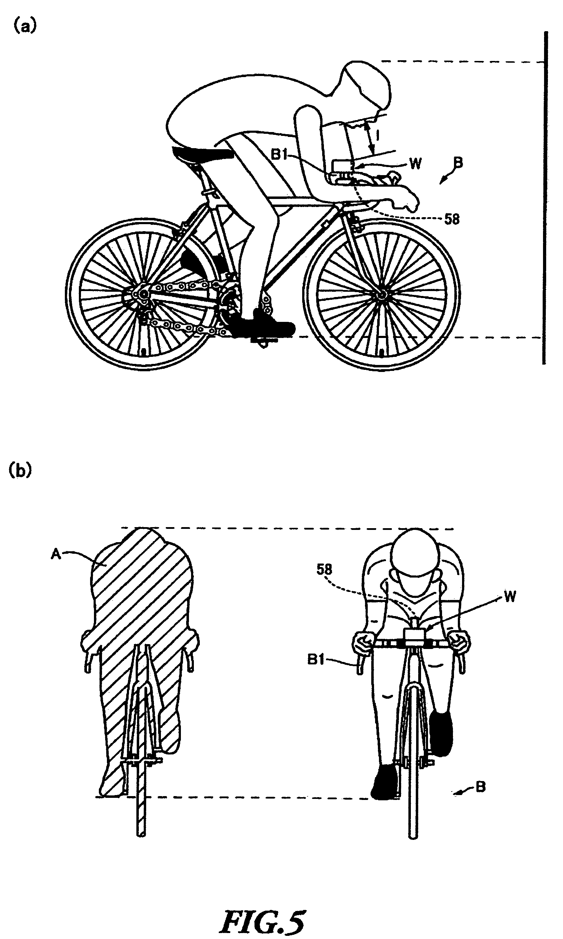

The distance measurement sensor 52 emits infrared radiation and receives the infrared radiation reflected from an object to be detected to measure the distance from the distance measurement sensor 52 to the object to be detected. The distance measurement sensor 52 is disposed before the display part 21 on the front face of the main body 7 so as to emit infrared radiation obliquely upward to the back side of the bicycle B. Here, as shown in FIG. 4(a) and FIG. 5(a), the direction of the infrared light emitted from the distance measurement sensor 52 is fixed such that the infrared light is directed to the movable part (e.g. the head or the breast) of the cyclist regardless of the posture of the cyclist.

The inclination sensor 53 detects the angle of inclination (°) of the bicycle B with the ground, and converts the measured value into gradient (%). The control device 6 calculates the average gradient of the route through which the bicycle B has run for a fixed period of time. The atmospheric pressure sensor 54 measures the atmospheric pressure around the bicycle B. The control device 6 calculates the altitude of the location of the bicycle B based on the atmospheric pressure measured by the atmospheric pressure sensor 54.

The temperature sensor 55 measures the temperature around the bicycle B (for example, the temperature of the location at which the bicycle B stops, or at which the bicycle B is running). The humidity sensor 56 measures the humidity around the bicycle B (for example, the humidity of the location at which the bicycle B stops, or at which the bicycle B is running).

The GPS sensor 57 is formed by a GPS chip antenna (not shown) and incorporated into a predetermined positioning system, such as a satellite positioning system, and therefore to obtain the current time as well as the positional information of the bicycle B. The GPS sensor 57 receives a signal transmitted from the satellite and transmits the signal to the control device 6.

Here, the above-described configuration of the sensor group 5 is merely an example. The data acquired by one senor may be acquired by the calculation based on the measured value that is measured by another sensor as long as the data can be acquired as a result. For example, the average of the inclination for a fixed section may be calculated without the inclination sensor 53, for example, by calculating the travel distance for a fixed period of time by the GPS sensor 57 and so forth, calculating the elevation difference for a fixed period of time required for the movement based on the elevation information calculated by the atmospheric pressure sensor 54, and using the travel distance and the altitude difference (elevation difference).

Moreover, altitude information may be obtained by the GPS sensor 57 instead of the humidity sensor 54, or by using three-dimensional map information including elevation information as map information.

The control device 6 includes: the CPU 61 responsible for control; a memory 62 that stores data indicating various information; an I/F 63 including an A/D converter that converts an analog electrical signal output from each sensor into a digital electrical signal and a serial I/F; an RTC 64 that continues to tick the current time; and an oscillating circuit 65 that functions to give a cue to perform timer interrupt processing described later. Here, the control device 6 is connected to the display control circuit 22, the input control circuit 32 and the communication control circuit 42 via respective buses.

The memory 62 is realized by an internal storage device such as a flash memory, a RAM, a ROM and a hard disk drive, or a nonvolatile external memory such as a USB memory and a flash memory card. The memory 62 temporarily or permanently stores the information obtained by the sensor group 5 of the power measurement apparatus W and the result of the computation performed by the CPU 61.

In addition, with the power measurement apparatus W, additional information such as map information is stored in the ROM in the memory 62 in advance, and this information is associated with the acquired or calculated data to create a bicycle map , and then the created bicycle map can be displayed on the display part 21.

A bandpass filter 14 shown in FIG. 3 constitutes the wind velocity measurement device 1 and is placed between the sound pressure sensor 12 and the corresponding A/D converter. With the present embodiment, the bandpass filter 14 is an op-amp bandpass filter that allows frequency band components of 1 to 4 kHz to pass through. In general, the frequencies of sound waves generated from the engine of a car or a bike (sound waves generated due to running) are distributed in the range of frequencies of 1 kHz or less, meanwhile the frequencies of the sounds of insects such as cicada are distributed in the range of frequencies of 4 kHz or more. In this way, even if the engine sound of a car and so forth and the sound of insects are detected by the sound pressure sensor 12, they are removed by the bandpass filter 14.

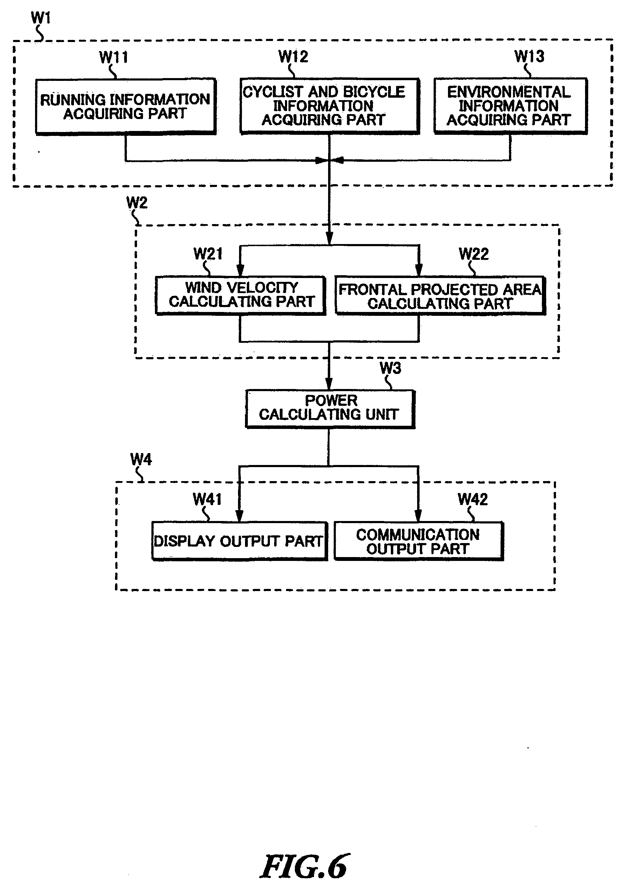

FIG. 6 is a control or functional diagram showing the power measurement apparatus W according to Embodiment 1 of the present invention. The power measurement apparatus W comprises: an information acquiring unit W1 including a running information acquiring part W11, a cyclist and bicycle information acquiring part W12 and an environmental information acquiring part W13; a variable calculating unit W2 including a wind velocity calculating part W21 and a frontal projected area calculating part W22; a power calculating unit W3; and an output unit W4 including a display output part W41 and a communication output part W42.

The running information acquiring part W11 has a function to acquire data of information on the running of the bicycle (hereinafter called "running information data"). The running information acquiring unit W1 is realized, for example, by the sensors such as the sound pressure sensor 12, the velocity sensor 51 and the GPS sensor 57, which measure the running information of the bicycle B, and the control device 6 that calculates and stores the running information, based on the running information data acquired by those sensors and predetermined data stored in the memory 62 in advance. Here, the predetermined data stored in the memory 62 includes the data stored in the ROM in advance and the data acquired by the input device 3 or the communication device 4 and stored in the RAM.

The cyclist and bicycle information acquiring part W12 has a function to acquire the data of the information on the body of the cyclist and also the information on the body of the bicycle (hereinafter called "cyclist and bicycle information data").

The cyclist and bicycle information acquiring part W12 is realized by: an input device 3 that outputs the cyclist and bicycle information data input by operating the input part 31 as an input information signal; and the control device 6 that recognizes input items selected by operating the input part 31 and stores the cyclist and bicycle information data received from the input device 3, in association with the recognized input items. Also the distance measurement sensor 52 that measures distance 1 between the bicycle B and the cyclist constitutes the cyclist and bicycle information acquiring part W12.

The environmental information acquiring part W13 has a function to acquire the data of information on the environment surrounding the bicycle B such as a natural environment and a geographical environment. The environmental information acquiring part W13 is realized by, among the sensor group 5, the inclination sensor 53, the atmospheric pressure sensor 54, the temperature sensor 55 and the humidity sensor 56 that measure environmental information.

The wind velocity calculating part W21 calculates the effective value of a sound pressure based on the data indicating the sound pressure acquired by the running information acquiring part W11, and calculates a wind velocity relative to the bicycle B based on the effective value of the sound pressure by using a predetermined transform equation. Here, the wind velocity calculating part W 21 is realized by the control device 6.

The frontal projected area calculating part W22 calculates frontal projected area A of a mobile object that is formed by the cyclist and the bicycle B integrated with one another (hereinafter called "mobile object") and that is influenced by the posture of the cyclist, based on the distance data indicating distance 1 acquired by the cyclist and bicycle information acquiring part W12, the data on the cyclist's height, the data on the type of the bicycle B, and the expression of the relation between the distance 1 and frontal projected area A stored in the ROM, which will be described later.

Frontal projected area A of the mobile object refers to an area that is projected on the vertical plane of the mobile object as shown in FIG. 4 and FIG. 5.

The power to resist the airflow moving relative to the bicycle B, which comes from the front side of the bicycle B is changed depending on the frontal projected area A. That is, the greater the frontal projected area A, the greater the power to resist the airflow relative to the bicycle B. Here, the frontal projected area calculating part W22 is realized by the control device 6.

The power calculating unit W3 calculates the power against air resistance by using data, such as the data on the wind velocity relative to the bicycle B, which is calculated by the wind velocity calculating part W21 (hereinafter called "wind velocity data"), and the data on the frontal projected area calculated by the frontal projected area calculating part W22 (hereinafter called "frontal projected area data"); calculates the power against grade resistance by using data such as the gradient data indicating the gradient of the ground acquired by the environmental information acquiring part W13; and calculates the power against rolling resistance by using data such as the velocity data indicating the velocity of the bicycle B acquired by the running information acquiring part W11. Then, the power calculating part W3 multiplies each power to calculate the entire power of the cyclist. Here, the power calculating unit W3 is realized by the control device 6.

The display output part W41 has a function to display a predetermined measured value or calculated value, or what the input part 31 is operated. The display output part W41 is realized by the display device 2. The communication output part W42 has a function to transmit predetermined data to an external device (not shown) and is realized by the communication device 4 and so forth. The communication output part W42 may have an antenna or a terminal for a cable and transmit data to a predetermined external device via the antenna or the cable. Moreover, the communication output part W42 may have a terminal for a USB memory and transmit data to the external device via the USB memory.

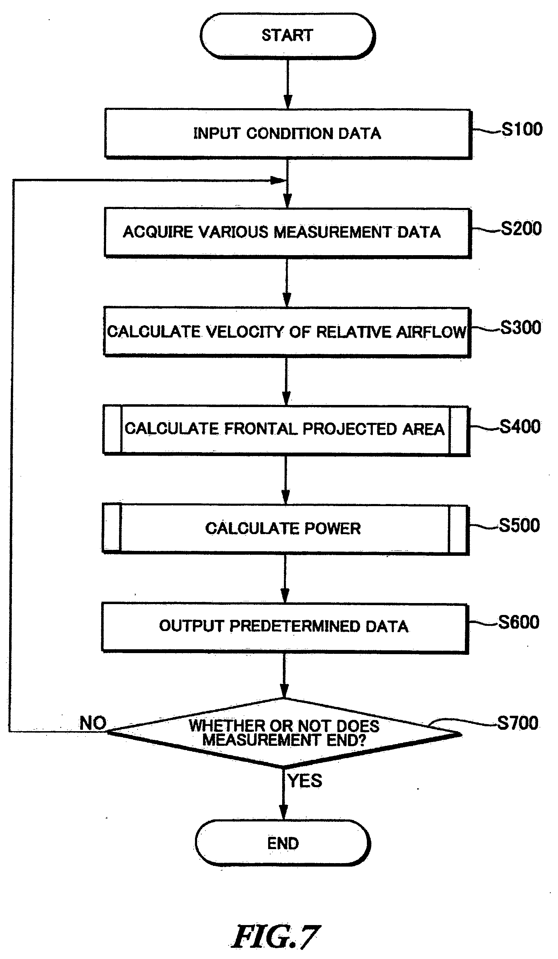

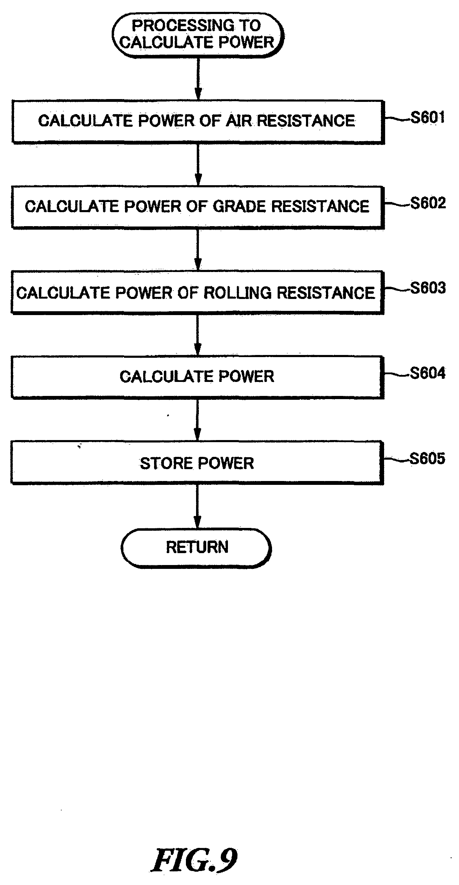

Next, with reference to FIG. 7 to FIG. 10, it will be explained that processing to measure the power of the cyclist on the bicycle B equipped with the power measurement apparatus W.

When the power supply of the power measurement apparatus W is turned on by using an on/off key provided in the input part 31 of the power measurement apparatus W, the control device 6 starts processing according to the flowchart shown in FIG. 7.

First, in step S100, when receiving data of predetermined information such as the height and weight of the cyclist, the type and the weight of the bicycle and the type and the air pressure of the tire, the CPU 61 stores the data in a predetermined area of the RAM in the memory 62. Then, when a measurement start key (not shown) provided in the input part 31 is appropriately operated, the CPU 61 determines the start of measurement, and moves the step to step S200.

In step S200, the CPU 61 acquires various data. That is, the CPU 61 stores various measurement data output from each sensor, in a predetermined area of the RAM in the memory 62. For example, the CPU 61 samples the velocity data indicating the velocity of the bicycle B that is output by the velocity sensor 51, the distance data indicating distance 1 that is output by the distance measurement sensor 52, the gradient data indicating the gradient of the ground that is output by the inclination sensor 53, and the sound pressure data indicating the sound pressure that is output by the sound pressure sensor 12, calculates the effective value of the measured value indicated by each data, and stores the effective value in a predetermined area of the RAM in the memory 62.

That is, the power measurement apparatus W acquires data on the velocity effective value indicating the effective value of the velocity of the bicycle B, data on the distance effective value indicating the effective value of distance 1, data on the gradient effective value indicating the effective value of the gradient of the ground, and data on the sound pressure effective value indicating the effective value of the sound pressure of the sound wave generated by the detection hole 13.

Here, if necessary, the power measurement apparatus W may calculate predetermined information based on the measurement data acquired by the sensors and the condition data acquired in step S100, and store the information in a predetermined area of the RAM in the memory 62.

In step S300, the CPU 61 calculates the velocity (relative velocity) of the airflow (relative wind) coming from the front of the bicycle B, which moves relative to the bicycle B. That is, the CPU 61 converts the effective value of a sound pressure into the wind velocity relative to the bicycle B, based on a predetermined transform equation which is stored in the ROM of the memory 62 in advance and in which the effective value of the sound pressure is associated with the wind velocity relative to the bicycle B, and also based on the data on the sound pressure effective value stored in the memory 62.

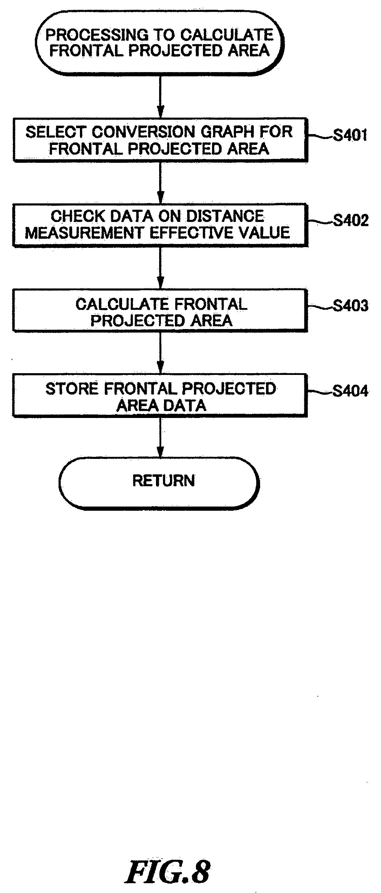

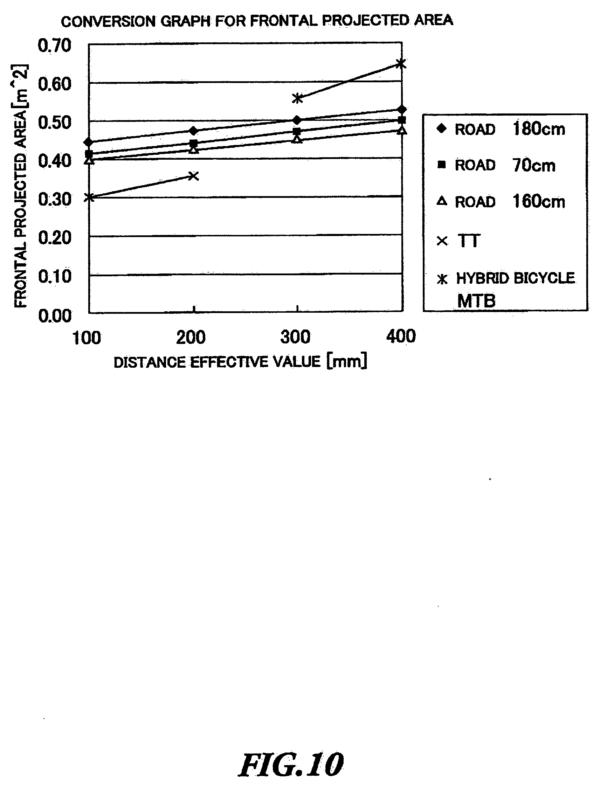

In step S400, the CPU 61 calculates frontal projected area A for the cyclist and the bicycle B. Here, processing to calculate this frontal projected area will be explained with reference to FIG. 8 and FIG. 10. First, in step S401, the CPU 61 selects a conversion graph for frontal projected area, from the data on the cyclist' height and the data on the bicycle type which have been acquired in step S100. The conversion graph for frontal projected area refers to a graph in which distance 1 from the distance measurement sensor 52 to the cyclist is associated with frontal projected area A of the mobile object.

Each pair of the cyclist' height and the bicycle type is set in this conversion graph for frontal projected area. It is because the posture of the cyclist on a bicycle varies depending on the type of the bicycle even if the cyclist is fixed. In addition, even if a bicycle is fixed, the frontal projected area basically varies depending on the cyclist' height.

For example, as shown in FIG. 10, "road race bike", "hybrid bike" an "time trial bike" are set as bicycle types. Then, the road race bicycle type is classified into 160 cm, 170 cm and 180 cm in height. Here, bicycle types, height types and combination thereof are not limited to the present embodiment.

In addition, as shown in FIG. 10, the set ranges for distance 1 for "hybrid bicycle" and "time trial bicycle" are narrower than for "road race bicycle", and each bar is partially missing. It is because the posture of a cyclist is nearly unchanged with these types. That is, the cyclist on a hybrid bicycle rarely runs in a forward bent position as shown in FIG. 5. Meanwhile, the cyclist on a time trial bicycle rarely runs in an approximate upright position. However, the set range for distance 1 for these types of bicycles may be the same as for the road race type.

In addition, as a method for inputting height data, either of two methods is possible: a method for directly inputting a height; or, a method for selecting a preset height. Moreover, as a method for selecting a graph, when a height is directly input, the CPU 61 selects a graph for the height that is the closest to the input height. Moreover, with the method for selecting a preset height, a graph is automatically selected at the time a height is input if the preset height is the same as the height set in the graph.

In step S402, the CPU 61 checks the data on the distance effective value stored in the memory 62. Then, in step S403, the CPU 61 calculates the frontal projected area A based on the graph selected in step S401 and the distance effective value checked in step S402. In step S404, the CPU 61 stores the data on the frontal projected area indicating the frontal projected area A in a predetermined area of the RAM in the memory 62 and ends the processing to calculate a frontal projected area. Here, in order to calculate the frontal projected area A, it may be possible to use a transform equation corresponding to a graph, instead of the graph.

In step S500, the CPU 61 calculates the power of the cyclist. Here, processing to calculate the power will be explained with reference to FIG. 10. First, in step S501, the CPU 61 calculates power Wa of the cyclist against air resistance (hereinafter called "air resistance power"). Air resistance power Wa is stored in the ROM of the memory 62 in advance and represented by the following Equation 1.

## Air resistance power Wa

$$= 0.5 \times \rho(kg/(m^3)) \times Cd \times A(m^2) \times (Vw((m/s))^3 \qquad ...(Equation\ 1).$$

Here, ρ represents atmospheric density. Atmospheric density data indicating the atmospheric density is stored in a predetermined area of the ROM in the memory 62 and set to, for example, "1.226". Cd represents air resistance coefficient. Air resistance coefficient data indicating the air resistance coefficient is stored in a predetermine area of the ROM in the memory 62 and set to, for example, "0.7". Vw represents the relative wind velocity. Wind velocity data indicating the relative wind has been calculated in step S300 and stored in the RAM of the memory 62.

In addition, A represents the frontal projected area. Frontal projected area data indicating the frontal projected area has been calculated in step S400 and stored in the RAM of the memory 62. In this way, the coefficients and variables constituting Equation 1 of air resistance power Wa is stored in the memory 62 as data. The CPU 61 plugs the data into Equation 1 to calculate air resistance power Wa and stores the air resistance power Wa in the RAM of the memory 62.

Next, in step S502, the CPU 61 calculates the power Ws of the cyclist against grade resistance (hereinafter called "grade resistance power"). The calculating formula for grade resistance power Ws is stored in the ROM of the memory 62 in advance and represented by the following Equation 2.

## Grade resistance power Ws

$$= m(kg) \times g(m/s^2)) \times \sin (arctan (\theta\ (\%)/100)) \times Vb\ (m/s)$$

$$....(Equation\ 2).$$

Here, m is the sum of the mass of the cyclist and the mass of the bicycle B and each mass is stored in the RAM of the memory 62 by operating the input part 31 in step S100. g represents acceleration of gravity and is stored in the ROM of the memory 62 in advance. The acceleration of gravity is set, for example, to "9.8". θ represents the gradient of the ground, which has been measured in step S200 and stored in the RAM of the memory 62.

In addition, Vb represents the velocity of the bicycle B, which has been acquired in step S200 and stored in the RAM of the memory 62. In this way, the coefficients and variables constituting Equation 2 of grade resistance power Ws are stored in the memory 62 as data. The CPU 61 plugs the data into Equation 2 to calculate the grade resistance power Ws and stores the grade resistance power Ws in the RAM of the memory 62.

Next, in step S503, the CPU 61 calculates power Wr of the cyclist against rolling resistance (hereinafter called "rolling resistance power"). The calculating formula for rolling resistance power Wr is stored in the ROM of the memory 62 in advance and represented by the following Equation 3.

## Rolling resistance power Wr

$$= m(kg) \times g((m/(s^2)) \times rolling\ resistance\ coefficient\ Crr \times Vb(m/s)$$

$$...(Equation\ 3).$$

Here, Crr represents rolling resistance coefficient, which is set in advance and stored in the ROM of the memory 62. The values m, g and Vb are the same as the above-description. In this way, the coefficients and variables constituting Equation 3 for the rolling resistance power are stored in the memory 62 as data. The CPU 61 plugs the data into Equation 3 to calculate the rolling resistance power and stores the rolling resistance power in the RAM of the memory 62.

In step S504, the CPU 61 multiplies the power calculated in each of step S501 to S503 to calculate the total of power W of the cyclist. In step S505, the CPU 61 stores power data indicating the total of power W in a predetermined area of the RAM in the memory 62 and ends the processing to calculate the power.

In step S600 in the main processing, the CPU 61 performs processing to output predetermined data. That is, the CPU 61 outputs predetermined data (e.g. power data, velocity data and travel distance data) to the display device 2, and displays the power, and the velocity and the travel distance of the bicycle B. Here, a target to which predetermined data is output, is not limited to the display device 2. For example, predetermined data set in advance may be output to an external device such as a PC installed in a home via the communication device 4.

In step S700, the CPU 61 determines whether or not to end the processing to measure the power. That is, the CPU 61 determines whether or not a measurement end signal to command to end the measurement has been input from the input part 31. If determining "Yes" in step S700, the CPU 61 ends the processing to calculate the power, and, on the other hand, if determining "No", the CPU 61 returns the step to step S200 and repeats steps S200 to S700.

As described above, with the power measurement apparatus W, the change in the frontal projected area of the mobile object, which constitutes the power of the cyclist on the bicycle B, is reflected in the power, so that it is possible to prevent any loss of the accuracy of power as a calculated value. Moreover, with the power measurement apparatus W, the type and the condition of the bicycle and also the information on the body of the cyclist, such as the height, are reflected in the power, and therefore can furthermore prevent loss of the accuracy.

Another Embodiment

With Embodiment 1, in order to calculate the frontal projected area A of a mobile object including the cyclist whose posture may significantly change, the distance between the cyclist and the distance measurement sensor 52 attached to the handle B1 is measured. However, the method for calculating the frontal projected area A is not limited to this.

For example, the positions of the hands gripping the handle, the angle of the elbow, the angle of the helmet, the height of the saddle, the positions of the pedals and so forth may be measured. In this way, the posture of the-cyclist, that is, a component that changes the frontal projected area A is measured to improve the accuracy of frontal projected area A. By this means, it is possible to also improve the accuracy in the calculation of the power of the cyclist.

For example, by measuring the positions of the pedals, it is possible to measure a change in the frontal projected area for the pedals of the bicycle B as well as a change in the frontal projected area for the legs of the cyclist. In addition, when the measurement by the distance measurement sensor 52 is determined as an error, that is, it may be presumed that infrared light is not reflected on the cyclist, the frontal projected area A may be set to the maximum value under the same condition, based on the determination that the cyclist is standing on the pedals.

Here, with Embodiment 1, although the mobile object is composed of the bicycle B and the cyclist, the mobile object may be constituted only by one of the bicycle B and the cyclist. However, it is preferred that the mobile object is constituted by the bicycle B and the cyclist, because the accuracy of the air resistance power Wa and the power W is improved when the mobile object is constituted by both of them.

In addition, with Embodiment 1, although atmospheric density $\rho$ is fixed as "1,293" to calculate the air resistance power Wa, air density $\rho$ may be calculated based on a temperature and an air pressure. By this means, it is possible to improve the accuracy of the air resistance power Wa. Here, in this case, the calculating formula for atmospheric density $\rho$ is represented, for example, by the following Equation 4.

$$\text{Atmospheric density } \rho \text{ (m)}$$
$$= 1.293 \text{ x atmospheric pressure} \div 1013 \text{ x } (1+(\text{temperature}/273))$$
$$\dots(\text{Equation 4}).$$

Moreover, with Embodiment 1, although the air resistance coefficient Cd is fixed to 0.7 to calculate the air resistance power Wa, the air resistance coefficient Cd may be calculated based on the type of clothes or the humidity. It is because the coefficient of skin friction between the atmospheric air and the cyclist varies depending on the material of the clothes or the humidity.

In this case, for example, in the processing to input condition data in step S100, it may be possible to input the material of the clothes from options by operating the input part 31. Here, the humidity is measured by the humidity sensor 56. In this way, by calculating the coefficient of skin friction between the atmospheric air and the cyclist, it is possible to improve the accuracy of air resistance power Wa, that is, the total of power W of the cyclist.

Moreover, with Embodiment 1, although the wind velocity calculating part W21, the frontal objected area calculating part W22 and the power calculating unit W3 are accommodated in the main body 7, they may also be accommodated in a mobile terminal such as a mobile phone, or a fixed terminal installed in a home. That is, a configuration is possible where the wind velocity calculating part W21, the frontal projected area calculating part W22 and the power calculating unit W 3 may be realized by application software stored in the ROM in a mobile terminal and so forth. In this case, for example, the sensor group 5 that measures data required to calculate power W is unitized (hereinafter, the unitized senor group is referred to as "sensor unit") and attached to the bicycle B.

Then, measurement data obtained by converting the measured value measured by each sensor of the sensor unit into an electrical signal is instantaneously transmitted to a mobile terminal and so forth. The mobile terminal and so forth calculates the relative wind velocity, the frontal projected area, the rolling resistance coefficient and the power. In this case, memories such as a RAM, a ROM and so forth may be mounted in the sensor unit, so that measurement data can be stored in the memories.

Moreover, another configuration is possible where the wind velocity part W21, the frontal projected area calculating part W22 and the power calculating unit W3 are mounted in a server connected to a fixed terminal and so forth, so that the server calculates the power. Here, the wind velocity calculating part W21, the frontal projected area calculating part W22 and the power calculating unit W3 install the frontal projected area A and the power W from a storage medium having a program stored thereon, or download this program via Internet.

With Embodiment 1, although the power measurement apparatus W is attached to the bicycle B and used in cycling, the use application of the power measurement apparatus W is not limited to cycling. For example, the power measurement apparatus W may be attached directly to the user and used in running, skiing and skating. In a case of running, although a sound wave that is not intended for detection is generated when the user sets foot on the ground, the time at which the user sets foot on the ground is detected by an acceleration sensor or an impact sensor, and the sound pressure data (unnecessary component) detected at that time is removed, so that it is possible to calculate the power accurately.

Meanwhile, in a case of skiing or skating, although when the traveling direction is changed, a sound wave that is not intended for detection is generated, a change in the travelling direction can be detected by an acceleration sensor. Therefore, it is possible to calculate the power accurately by removing the acceleration data (unnecessary component) when the travelling direction is changed.

Moreover, as a method for calculating a frontal projected area, it may be possible to adopt image processing to calculate a frontal projected area, instead of the method as in Embodiment 1 that calculates a frontal projected area based on the distance between the distance measurement sensor and the cyclist. For example, by taking an image of the cyclist with a digital camera attached to the bicycle and by using image data obtained by taking the image, it is possible to calculate the frontal projected area of the cyclist based on the area of the cyclist on the image. Here, in this case, the digital camera may be incorporated into the above-described sensor unit.

List of Reference Signs

| 1 | = wind velocity detecting device |
|---|---|
| 2 | = display device |
| 3 | = input device |
| 4 | = communication device |
| 5 | = sensor group |
| 6 | = control device |
| 7 | = main body |
| 11A | = inflow port |
| 11B | = outflow port |
| 12 | = sound pressure sensor |
| 13 | = detection port |
| 21 | = display part |
| 22 | = display control circuit |
| 31 | = input part |
| 32 | = input control circuit |
| 41 | = communication interface |
| 42 | = communication control circuit |
| 61 | = CPU |
| 62 | = memory |
| 63 | = A/D converter |
| 64 | = RTC |
| 65 | = oscillating circuit |
| B | = bicycle |
| B1 | = handle |
| W | = power measurement apparatus |
| W1 | = information acquiring unit |
| W2 | = variable calculating unit |
| W3 | = power calculating unit |
| W4 | = output unit |
| W11 | = running information acquiring part |
| W12 | = cyclist and bicycle information acquiring part |
| W13 | = environmental information acquiring part |
| W21 | = wind velocity calculating part |

W22 = frontal projected area calculating part
W41 = display output part
W42 = communication output part

**Claims**

1. A power measurement apparatus (W) configured to measure power of work done on a bicycle comprising:

    - an input part (W1)configured to input at least one of information on a type of the bicycle and information on a body of a cyclist on the bicycle;

    **characterized in that**

    - a distance measuring part (W12) is configured to measure a distance from a predetermined position of the bicycle to the cyclist;
    - a frontal projected area calculating part (W22) is configured to calculate a frontal projected area of a movable object that includes at least the cyclist and moves on a ground, based on the information inputted by the input part (W1) and the distance measured by the distance measuring part (W12); and
    - a power measuring part (W3) is configured to measure a power of work done on the bicycle by calculating air resistance against the movable object, based on a wind velocity relative to the bicycle and the frontal projected area calculated by the frontal projected area calculating part (W22).

2. A program that allows a computer to function as:

    - an input means for inputting at least one of information on a type of the bicycle and information on a body of a cyclist on the bicycle;

    **characterized in that** said program allows said computer to function further as:

    - a frontal projected area calculating means (W22) for calculating a frontal projected area of a movable object that includes at least the cyclist and moves on a ground, based on the information inputted by the input part (W1) and the distance from a predetermined position of the bicycle to the cyclist; and
    - a power measuring means (W3) for measuring power of work done on the bicycle by calculating air resistance against the movable object, based on a wind velocity relative to the bicycle and the frontal projected area calculated by the frontal projected area calculating means (W22).

3. A computer-readable storage medium,
    having a program according to claim 2 stored therein.

**Patentansprüche**

1. Leistungsmessvorrichtung (W), die für die Messung von Arbeitsleistung konfiguriert ist, die auf einem Fahrrad verrichtet wird,
    wobei die Vorrichtung Folgendes aufweist:

    - eine Eingabeeinheit (W1), die konfiguriert ist, um zumindest eine Information einzugeben von einer Information über einen Typ des Fahrzeugs und Information über den Körper eines Radfahrers auf dem Fahrrad;
    **dadurch gekennzeichnet,**
    **dass** eine Distanzmesseinheit (W12) konfiguriert ist, um eine Distanz von einer vorbestimmten Position des Fahrrads zu dem Radfahrer zu messen; dass eine Recheneinheit (W22) für einen frontal vorstehenden Bereich konfiguriert ist, um einen frontal vorstehenden Bereich eines beweglichen Objektes, das zumindest den Radfahrer umfasst und das sich auf dem Boden bewegt, berechnet, und zwar auf der Basis von der Information, die von der Eingabeeinheit (W1) eingegeben wird, und der Distanz, die mit der Distanzmesseinheit (W12) gemessen wird;
    und **dass** eine Leistungsmesseinheit (W3) konfiguriert ist, um eine Arbeitsleistung zu messen, die auf dem Fahrrad verrichtet wird, und zwar durch Berechnen des Luftwiderstandes gegenüber dem beweglichen Objekt, und zwar auf der Basis einer Windgeschwindigkeit relativ zu dem Fahrrad und dem frontal vorstehenden Bereich, der mit der Recheneinheit (W22) für den frontal vorstehenden Bereich berechnet wird.

2. Programm, das es einem Computer ermöglicht, seine Funktion zu erfüllen als:

- eine Eingabeeinheit zum Eingeben von mindestens einer Information von Information über einen Typ des Fahrrads und Information über den Körper eines Radfahrers auf dem Fahrrad;
**dadurch gekennzeichnet,**
**dass** das Programm es dem Computer ermöglicht, ferner folgende Funktionen zu erfüllen:
- eine Recheneinheit (W22) für einen frontal vorstehenden Bereich, um einen frontal vorstehenden Bereich eines beweglichen Objektes zu berechnen, das zumindest den Radfahrer umfasst und das sich auf dem Boden bewegt, und zwar auf der Basis von der Information, die mit der Eingabeeinheit (W1) eingegeben wird, und der Distanz von einer vorbestimmten Position des Fahrrads zu dem Radfahrer; und
- eine Leistungsmesseinheit (W3), um eine Arbeitsleitung zu messen, die auf dem Fahrrad verrichtet wird, und zwar durch Berechnen des Luftwiderstandes gegenüber dem beweglichen Objekt, und zwar auf der Basis einer Windgeschwindigkeit relativ zu dem Fahrrad und dem frontal vorstehenden Bereich, der mit der Recheneinheit (W22) für den frontal vorstehenden Bereich berechnet wird.

3. Computerlesbares Speichermedium,
in welchem ein Programm gemäß Anspruch 2 abgespeichert ist.

**Revendications**

1. Appareil de mesure de puissance (W) configuré pour mesurer la puissance d'un travail fourni sur une bicyclette, comprenant:

- une partie d'entrée (W1) configurée pour entrer au moins une information parmi une information sur un type de la bicyclette et une information sur un corps d'un cycliste sur la bicyclette;

**caractérisé en ce que**

- une partie de mesure de distance (W12) est configurée pour mesurer une distance depuis une position prédéterminée de la bicyclette jusqu'au cycliste;
- une partie de calcul de superficie en projection frontale (W22) est configurée pour calculer une superficie en projection frontale d'un objet mobile qui inclut au moins le cycliste et qui se déplace sur un sol, en se basant sur l'information entrée par la partie d'entrée (W1) et sur la distance mesurée par la partie de mesure de distance (W12); et
- une partie de mesure de puissance (W3) est configurée pour mesurer une puissance d'un travail fourni sur la bicyclette en calculant une résistance à l'air à l'encontre de l'objet mobile, sur la base d'une vitesse du vent par rapport à la bicyclette et de la superficie en projection frontale calculée par la partie de calcul de superficie en projection frontale (W22).

2. Programme qui permet un ordinateur de fonctionner comme:

- un moyen d'entrée pour entrer au moins une information parmi une information sur un type de bicyclette et une information sur un corps d'un cycliste sur la bicyclette;

**caractérisé en ce que**
ledit programme permet audit ordinateur de fonctionner en outre comme:

- un moyen de calcul de superficie en projection frontale (W22) pour calculer une superficie en projection frontale d'un objet mobile qui inclut au moins le cycliste et qui se déplace sur un sol, en se basant sur l'information entrée par la partie d'entrée (W1) et sur la distance depuis une position prédéterminée de la bicyclette jusqu'au cycliste; et
- un moyen de mesure de puissance (W3) pour mesurer la puissance de travail fourni sur la bicyclette, en calculant la résistance à l'air à l'encontre de l'objet mobile, sur la base d'une vitesse du vent par rapport à la bicyclette et de la superficie en projection frontale calculée par le moyen de calcul de superficie en projection frontale (W22).

3. Support de stockage lisible à l'ordinateur,
   ayant un programme selon la revendication 2 stocké dans lui-même.

(a)

(b)

*FIG.1*

**FIG.2**

**FIG.3**

(a)

(b)

*FIG.4*

(a)

(b)

*FIG.5*

W1

W11 | W12 | W13

RUNNING INFORMATION ACQUIRING PART | CYCLIST AND BICYCLE INFORMATION ACQUIRING PART | ENVIRONMENTAL INFORMATION ACQUIRING PART

W2

W21 | W22

WIND VELOCITY CALCULATING PART | FRONTAL PROJECTED AREA CALCULATING PART

W3

POWER CALCULATING UNIT

W4

W41 | W42

DISPLAY OUTPUT PART | COMMUNICATION OUTPUT PART

*FIG.6*

START

INPUT CONDITION DATA — S100

ACQUIRE VARIOUS MEASUREMENT DATA — S200

CALCULATE VELOCITY OF RELATIVE AIRFLOW — S300

CALCULATE FRONTAL PROJECTED AREA — S400

CALCULATE POWER — S500

OUTPUT PREDETERMINED DATA — S600

NO — WHETHER OR NOT DOES MEASUREMENT END? — S700

YES

END

*FIG.7*

```
          PROCESSING TO CALCULATE
           FRONTAL PROJECTED AREA

                      │
                      ▼

          SELECT CONVERSION GRAPH FOR        S401
             FRONTAL PROJECTED AREA

                      │
                      ▼

            CHECK DATA ON DISTANCE           S402
         MEASUREMENT EFFECTIVE VALUE

                      │
                      ▼

              CALCULATE FRONTAL              S403
               PROJECTED AREA

                      │
                      ▼

          STORE FRONTAL PROJECTED            S404
                 AREA DATA

                      │
                      ▼

                   RETURN
```

# FIG.8

PROCESSING TO
CALCULATE POWER

CALCULATE POWER OF AIR RESISTANCE —S601

CALCULATE POWER OF GRADE RESISTANCE —S602

CALCULATE POWER OF ROLLING RESISTANCE —S603

CALCULATE POWER —S604

STORE POWER —S605

RETURN

*FIG.9*

FIG.10

**EP 2 583 887 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7151620 A
- WO 02081257 A2, VOSS DARELL